(19) Europäisches Patentamt
European Patent Office
Office européen des brevets

(11) **EP 1 357 861 B1**

(12) **EUROPÄISCHE PATENTSCHRIFT**

(45) Veröffentlichungstag und Bekanntmachung des Hinweises auf die Patenterteilung:
**06.05.2009 Patentblatt 2009/19**

(21) Anmeldenummer: **02716734.5**

(22) Anmeldetag: **06.02.2002**

(51) Int Cl.:
**A61F 2/18** (2006.01)     **A61M 3/02** (2006.01)
**A61M 31/00** (2006.01)

(86) Internationale Anmeldenummer:
**PCT/EP2002/001228**

(87) Internationale Veröffentlichungsnummer:
**WO 2002/062269 (15.08.2002 Gazette 2002/33)**

(54) **PLATZHALTER FÜR WIRKSTOFFFREIGABE IN DER NASENNEBENHÖHLE**

SPACING DEVICE FOR RELEASING ACTIVE SUBSTANCES IN THE PARANASAL SINUS

DISPOSITIF DE MAINTIEN D'ESPACE POUR LIBERATION D'UN PRINCIPE ACTIF DANS LE SINUS NASAL

(84) Benannte Vertragsstaaten:
**AT BE CH CY DE DK ES FI FR GB GR IE IT LI LU MC NL PT SE TR**

(30) Priorität: **06.02.2001 DE 10105592**

(43) Veröffentlichungstag der Anmeldung:
**05.11.2003 Patentblatt 2003/45**

(73) Patentinhaber: **Acclarent, Inc.**
**Menlo Park, CA 94025 (US)**

(72) Erfinder:
• **Göpferich, Achim, Prof. Dr.**
**93161 Sinzing (DE)**

• **Hosemann, Werner**
**17487 Greifswald (DE)**

(74) Vertreter: **Coates, Ian Harold et al**
**Scott & York**
**Intellectual Property Limited**
**45 Grosvenor Road**
**St. Albans**
**Hertfordshire AL1 3AW (GB)**

(56) Entgegenhaltungen:
EP-A- 0 585 757          WO-A-00/53252
CH-A- 668 188            US-A- 4 755 171
US-A- 5 139 832          US-A- 5 601 594
US-A- 5 693 065          US-A- 5 827 224

**Beschreibung**

Die vorliegende Erfindung betrifft einen Platzhalter (Stent) zum Einsatz in Fensterungen der Nasennebenhöhle.

An einer chronischen Schleimhautentzündung der Nasennebenhöhlen leiden etwa 5 % unserer Bevölkerung. Bei jedem fünften Patienten kommt es im Verlauf einer derartigen Entzündung zur Ausbildung von Nasenpolypen. Treten entsprechende Beschwerden auf und bleibt ein medikamentöser Behandlungsversuch erfolglos, so wird die chronische Sinusitis operativ angegangen.

Das Nebenhöhlensystem besteht aus einer Reihe luftgefüllter und mit Schleimhaut ausgekleideter Hohlräume. Eine wichtige Rolle beim Zustandekommen einer chronischen Sinusitis spielt die Unterbrechung der natürlichen Sekretdrainage aus den entfernt liegenden Nebenhöhlenabschnitten und die Aufhebung der natürlichen Belüftung. Dementsprechend werden die entfernt liegenden, erkrankten Nebenhöhlen-Abschnitte im Rahmen sanierender Eingriffe durch neu geschaffene oder erweiterte Zugänge ("Fenster") wieder belüftet. Nach Ausheilung stellt sich auch die natürliche Sekretdrainage dieser wiederbelüfteten Kompartimente erneut ein.

Die chirurgische Therapie der chronischen Sinusitis hat sich nach Einführung moderner optischer Hilfen (starre Endoskope, Mikroskop) gewandelt. Im Vordergrund steht heute die "minimal-invasive" Ausräumung ausschließlich derjenigen Schleimhautanteile, die eine offensichtlich irreversible Veränderung durch den Entzündungsprozess erfahren haben. Andere, reversibel veränderte oder gar nicht betroffene Schleimhautareale werden möglichst weitgehend geschont (Hosemann WG, Weber RK, Keerl RE, Lund VJ: Minimally invasive endonasal sinus surgery. Thieme, Stuttgart, New York 2000).

Ist die Stirnhöhlenschleimhaut an dem entzündlichen Umbau der Nebenhöhlen beteiligt, so erfolgt eine operative Fensterung zur Nase hin. Sie wird mit einem speziellen Instrumentarium (gebogenen scharfen Löffeln, speziellen Stanzen, Bohrern) ausgeführt. Durch die routinemäßige 'Fensterung' der Stirnhöhle werden Zugänge von etwa 5 bis maximal 10 mm Durchmesser hergestellt. Im Rahmen der Wundheilung verengen sich diese Zugänge um etwa 1,5 mm.

Liegen bestimmte gesundheitliche Faktoren wie z.B. eine Analgetika-Intoleranz vor, so muss man mit einer überproportionalen Tendenz zur narbigen Verengung rechnen (Hosemann W, Th. Kühnel, P. Held, W. Wagner, A. Felderhoff: Endonasal frontal sinusotomy in surgical management of chronic sinusitis - a critical evaluation. Am. J. Rhinology 11: 1-9 (1997)). Für solche Fälle wird angeraten, den operativen Zugang vorsorglich maximal zu gestalten. Diese "erweiterte Stirnhöhlenchirurgie" wird in bestimmte Typen untergliedert (Draf W: Endonasal micro-endoscopic frontal sinus surgery: the Fulda concept. Op Tech Otolaryngol Head Neck Surg 2: 234-240 (1991); May M, Schaitkin B: Frontal sinus surgery: endonasal drainage instead of an external osteoplastic approach. Op Tech Otolaryngo Head Neck Surg 6: 184-192 (1995)).

Wie ausgeführt, verengt sich das Neo-Ostium zur Stirnhöhle erfahrungsgemäß mehr oder minder ein. Um dieser narbigen Stenosierung vorzubeugen, wurde schon zu Beginn des letzten Jahrhunderts, d.h. lange vor Einführung der minimal-invasiven, endoskopischen Chirurgie vorgeschlagen, einen Platzhalter (stent) einzusetzen. Diese Platzhalter besaßen meist die Form eines Röhrchens, sie waren aus unterschiedlichen Materialien: anfangs wurde gewalztes oder in Drähten geflochtenes Metall eingesetzt (Fletscher Ingals E: New operation and instruments for draining the frontal sinus. Ann Otol Rhinol Laryngol 14: 515-519 (1905), Good RH: An intranasal method for opening the frontal sinus establishing the largest possible drainage. Laryngoscope 18: 266-274 (1908)). In den letzten zwei Jahrzehnten wurden Silikonröhrchen bevorzugt (Stammberger H: Komplikationen entzündlicher Nasennebenhöhlenerkrankungen einschließlich iatrogen bedingter Komplikationen. Eur Arch Oto-Rhino-Laryngol Suppl 1993/I: 61-186).

Die Erfahrung mit Platzhaltern zur Stabilisierung des neu geschaffenen Stirnhöhlenzuganges waren jedoch nicht immer ermutigend, von einzelnen Mitteilungen abgesehen (Jacobs JB: 100 years of frontal sinus surgery. Laryngoscope 107: 1-36 (1997); Weber, R, W. Hosemann, W. Draf, R. Keerl, B. Schick, S. Schinzel: Endonasale Stirnhöhlenchirurgie mit Langzeiteinlage eines Platzhalters. Laryngol. Rhinol. Otol. 76: 728-734 (1997)).

Unklar blieb zunächst, wie lange ein derartiger Platzhalter im Operationsgebiet benötigt würde. Aus Tierversuchen zur Wundheilung wurde deutlich, dass mit einer narbigen Einengung des Stirnhöhlenzuganges über einen Zeitraum von mindestens drei Monaten postoperativ gerechnet werden muss (Hosemann, M.E. Wigand, U. Göde, F. Länger, I. Dunker: Normal wound healing of the paranasal sinuses - clinical and experimental investigations. Eur. Arch. Otorhinolaryngol. 248: 390-394 (1991)). Dementsprechend müsste der Platzhalter über 8 bis 12 Wochen eingesetzt werden. Auch bei korrekter Liegedauer wird ein Platzhalter die unerwünschte narbige Einengung des Stirnhöhlenzuganges oft nur zeitlich verzögern und evtl. an Umfang vermindern, ohne ihn komplett verhindern zu können. Hier müsste eine ergänzende medikamentöse Behandlung zur Reduktion überschießender Wundreaktionen erfolgen.

Nach derzeitigem Kenntnisstand über die Wundheilungsvorgänge in der Nase scheint die Gabe von Arzneistoffen, wie z.B. Kortikoiden in der Lage zu sein, dieser Tendenz der regenerierenden Schleimhaut zur narbigen Striktur des Stirnhöhlen-Nasen-Ganges mit einer gewissen Verlässlichkeit entgegenzuwirken (Hosemann, M.E. Wigand, U. Göde, F. Länger, I. Dunker: Normal wound healing of the paranasal sinuses - clinical and experimental investigations. Eur. Arch. Otorhinolaryngol. 248: 390-394 (1991); Hosemann W, Göde U, Länger F, Wigand ME: Experimentelle Untersuchungen zur Wundheilung in den Nasennebenhöhlen. II. Spontaner Wundschluss und medikamentöse Effekte im standardisierten Wundmodell. HNO 39: 48-54 (1991); Hosemann W, Kühnel Th, Allert MH: Weiterbehandlung nach Nasennebenhöhleneingriffen, Teil 2: Therapeutische Maßnahmen. HNO aktuell 7: 291-302 (1999)).

Unglücklicherweise gelangen konventionelle Arzneiformen wie Salben oder Sprays bei routinemäßiger Anwendung nicht in den Problembereich des Überganges von Stirnhöhle und Nase (Prince MEP, Lemckert RJ: Analysis of the intranasal distribution of ointment. J Otolaryngol 26: 357-360 (1997); Weber R, Keerl R, Radziwill R, Schick B, Jaspersen D, Dshambazov K, Mlynski G, Draf W: Videoendoscopic analysis of nasal steroid distribution. Rhinology 37: 69-73 (1999)).

Eine postoperative systemische Gabe von Kortikoiden ist in der Rhinochirurgie zwar durchaus gebräuchlich (Bumm P: Hals-Nasen-Ohrenkrankheiten. In: Kaiser H, Kley HK (Hrsg.) Cortisontherapie, Corticoide in Klinik und Praxis. Thieme, Stuttgart 1992, pp 390 401), die Behandlungs-Schemata erstrecken sich jedoch meist nicht über den geforderten Zeitraum von 8 Wochen. Bei längerfristigen systemischen Kortikoid-Gaben ist darüber hinaus vermehrt mit Nebenwirkungen der Behandlung zu rechnen.

Die vorstehend beschriebenen Probleme zeigen den Bedarf an Systemen, die Wirkstoffe wie z.B. Kortikoide über längere Zeit direkt an den Operationsort kontrolliert abgeben können.

Für die kontrollierte Freigabe von Arzneistoffen sind eine Reihe von Systemen vorgeschlagen werden wie z.B. Implantate aus arzneistoffbeladenen Polymeren. So beschreibt US 5,633,000, Implantate zur Freigabe von Schmerzmitteln. Die dort verwendeten Polymere geben den Wirkstoff über Diffusion frei. US 5,019,372 beschreibt, dass sich diese Freigabe, über Einarbeitung magnetischer Partikel und über das Anlegen von wechselnden Magnetfeldern, modulieren lässt. Gestaltet man diese Formkörper mit entsprechend definierter Geometrie aus, dann lässt sich die Freigabe der Wirkstoffe so optimieren, dass sie mit konstanter Geschwindigkeit über den Anwendungszeitraum freigesetzt werden (US 4,803,076).

Unter den Polymeren, die für solche Anwendungen eingesetzt werden, finden sich neben bioabbaubaren auch nicht bioabbaubare Materialien d.h., solche die sich bei Kontakt mit Körperflüssigkeiten nicht zersetzen. Beispiele für solche Polymere sind Silikon, Polyacrylate und Ethylenvinylacetat-Copolymer (US 4,069,307). Insbesondere die letzte Polymergruppe wurde für eine Reihe von Systemen zur kontrollierten Freigabe an Wirkstoffen eingesetzt.

So beschreibt US 3,393,073 ein sogenanntes Reservoirsystem, welches aus einem Arzneistoffvorrat besteht, der von einer Polymerhülle, welche die Freigabegeschwindigkeit des Arzneistoffs reguliert, umgeben ist. Solche Systeme wurden erfolgreich eingesetzt für die Entwicklung von ‚Intra-Uterine-Devices', die den Wirkstoff im Uterus freisetzen (US 3,967,618 und US 4,016,251) und für die Herstellung therapeutischer Systeme, die Arzneistoffe am Auge freisetzen (US 4,052,505).

Als Trägersysteme mit mikroporöser Membran, welche die Wirkstoffabgabe kontrollieren, wurden solche Systeme auch für das Einbringen in verschiedene Körperhöhlen beschrieben, so z.B. das Ohr, die Nase oder auch das Rektum (US 3,948,254).

Im Bereich der "Stents" für die Behandlung der Nasennebenhöhlen sind zwar solche aus Kunststoff beschrieben, wie z.B. in US 5,693,065 oder US 5,336,163. US 5,693,065 beschreibt einen Stent für den Nasenbereich aus Siliconkautschuk, der einen zylinderförmigen Schaft aufweist, dessen in die Nase einzuführendes vordere Ende speerspitzenförmig gestaltet ist, wobei die Basis der Spitze, die mit dem Schaft verbunden ist, einen breiteren Durchmesser als der Schaft hat. Die Spitze ist vorne geschlossen und weist seitlich Rippen mit Schlitzen dazwischen auf, wobei sich die Rippen im eingeführten Zustand ausweiten und so den Halt des Stents im Nasenbereich gewährleisten.

Als Abmessungen für den Schaft ist ein Außendurchmesser von 4 mm (0,157 Inch) und ein innerer Durchmesser von 3 mm ( 0,118 Inch) angegeben. Der feste Sitz des Stents im Nasengang wird jedoch allein durch das Aufspreizen der speerförmigen Spitze gewährleistet.

US 5,336,163 betrifft einen Stent für den Nasenbereich, der aus einem porösen Material gebildet ist und eine nicht haftende, allenfalls geringfügig poröse äußere Oberfläche aufweist. Dabei wird der Stent aus einem Material gebildet, das bei Kontakt mit Flüssigkeit expandiert.

US 5,601,594 beschreibt einen Stent zum Einführen in eine Nasenöffnung, wobei der Stent eine gebogene Form aufweist und aus einem komprimierbaren Material gebildet ist.

Allerdings handelt es sich dabei um Systeme, die arzneistofffrei sind und deren Wirkung darauf abzielt, allein durch physikalisch/ mechanische Effekte Zugänge zur Stirnhöhle offen zu halten.

Trotz dieses Fortschritts im Bereich der kontrollierten Freigabe von Wirkstoffen, gibt es bislang keine Hinweise darauf, dass diese Technologie für die postoperative Versorgung der Nebenhöhlensysteme nach minimal invasiver Ausräumung einsetzbar wäre. Zwar kennt man sogenannte "Stents", die eine Gewebeneubildung verhindern, allerdings sind diese ausschließlich für die Behandlung von Blutgefäßen beschrieben und dementsprechend auf andere biologische Bedürfnisse zugeschnitten.

US Patent 5,980,551 beschreibt einen Stent für Blutgefäße, wobei der Stent eine innere Stützstruktur aufweist, die aus einem Draht gebildet sein kann, und diese Stützstruktur von einem bioabbaubaren resorbierbaren Substrat umgeben ist. In dieses Substrat können biologisch aktive Mikroteilchen eingelagert werden, die kontrolliert Wirkstoffe freisetzen.

Stents zur Unterdrückung der Restenose von Koronararterien weisen Designmerkmale auf, die sie vom Gegenstand der Erfindung deutlich unterscheiden und sie damit auch für die Anwendung in der Stirnhöhle ungeeignet machen.

In vielen Fällen erfordern die 'Koronarstents' zudem Applikationshilfen. In US 6,080,190 and US 5,843,089 sind solche Applikationshilfen in Kombination mit einem Stent beschrieben.

Ein gravierendes Problem von Koronarstents zur Freigabe von Wirkstoffen ist der Aufbau des Arzneistofffreigabesystems. Koronarstents bestehen zumeist aus einem Stentkörper wie z.B. einem Drahtgeflecht das mit Arzneistofftragenden Polymeren überzogen ist oder in dünne Polymerfilme eingehüllt wird (US 5,824,048, US 5,700,286, US 5,837,313, US 5,679,400). Die mechanische Stabilität dieser Stents ist auf die Bedürfnisse von Arterien zugeschnitten und macht sie für die Anwendung in der Nasenhöhle ungeeignet, da sie mechanisch nicht stabil genug sind.

Koronarstents sind rotationssymmetrische Hohlkörper und haben vorzugsweise die Geometrie eines Hohlzylinders. Sie lassen sich deshalb in einer Fensterung der Nasennebenhöhle z. B. nicht über Verdikkungen am Zylinderende fixieren. Darüber hinaus ist eine Fensterung der Nasennebenhöhle üblicherweise nicht gleichförmig rund sondern mehr oder weniger unregelmäßig, was zusätzliche Probleme in Bezug auf die Verankerung schafft. Generell dürfen Konronarstents keine großen Wanddicken aufweisen, um den Blutfluss nicht zu behindern.

Darüber hinaus unterscheiden sich Koronarstents von Platzhaltern für Nasennebenhöhlen durch ihre Funktion. Der Koronarstent soll in vielen Fällen das Gefäß aufdehnen. Der Stirnhöhlen-Platzhalter hingegen wird in einen operativ angelegten Gang eingelegt, der knöcherne (stabile) Wände besitzt. Dieser Gang wurde operativ neu gestaltet, das Herzkranzgefäß dagegen wird als Rohr belassen, jedoch aufgedehnt.

Ein Koronar-Stent gilt als permanentes Implantat, er wird vom Körper komplett aufgenommen. Der Stirnhöhlen-Platzhalter wird hingegen nach einer Zeit von etwa 8 Wochen entnommen.

Der Koronarstent wird vom Körper komplett aufgenommen. Im Inneren des Koronarstents fließt Blut, die Wand wird im günstigen Fall von körpereigenen Zellen (Endothelzellen) besiedelt. Beim Stirnhöhlen-Platzhalter ist eine komplette Aufnahme in den Körper nicht erwünscht. Im Inneren des Stirnhöhlen-Platzhalters soll Sekret von der Schleimhautoberfläche abfließen und gleichzeitig eine Belüftung sichergestellt sein. Eine Besiedelung der Innenseite des Platzhalters mit körpereigenen Zellen ist weder vorgesehen noch erwünscht.

Auf der anderen Seite soll sich die Schleimhaut an der Außenseite des Stirnhöhlen-Platzhalters ausbreiten. Auf diese Weise soll nach dem Entfernen des Platzhalters ein mit intakter Schleimhaut ausgekleideter Gang zurückbleiben.

Ein Problem ist beim Koronarstent die Gerinnselbildung mit der Gefahr eines Verschlusses, welche durch die Gabe von speziellen Medikamenten unterdrückt werden muss. Der Stirnhöhlen-Platzhalter zwingt nicht zur Gabe spezieller Medikamente.

Es ist vorgeschlagen worden, medizinische Vorrichtungen, die in einem Körper verwendet werden, aus einem wirkstoffbeladenen Material anzufertigen oder damit zu überziehen.

In der WO 96/29071 werden medizinische Vorrichtungen wie Katheter oder Stents beschrieben, auf deren Oberfläche antibakterielle Mittel aufgebracht sind, wobei die antibakteriellen Mittel auf der Oberfläche aufgrund von Adhäsionskräften anhaften ohne dass weitere Hilfsmittel erforderlich sind.

In der WO 92/15286 wird allgemein vorgeschlagen, medizinische Vorrichtungen aus einem arzneistoffbeladenen Polymer zu bilden oder mit einem Überzug davon zu versehen, wobei auch Stents zum Einsatz im Nasenbereich genannt sind, ohne nähere Angaben zur Ausgestaltung eines derartigen Stents.

Es war Aufgabe der vorliegenden Erfindung einen Platzhalter zur Verfügung zu stellen, der insbesondere zum Einsatz in die Nasennebenhöhle geeignet ist und nicht nur eine ausreichende Stabilität sowie feste Halterung aufweist, sondern gleichzeitig vor Ort einen gewünschten Wirkstoff kontrolliert freisetzen kann, wobei eine kontrollierte Freisetzung der erforderlichen Menge Wirkstoff mit dem gewünschten zeitlichen Verlauf auch über einen für die Behandlung ausreichend langen Zeitraum gewährleistet ist.

Zudem ermöglicht der erfindungsgemäße Platzhalter die Aufnahme und Speicherung einer ausreichend großen Menge an Wirkstoff, ohne Beeinträchtigung der kontrollierten Freisetzung aufgrund von Wechselwirkungen der Wirkstoffanteile untereinander.

Erfindungsgemäß wird diese Aufgabe gelöst durch einen Platzhalter wie er im unabhängigen Anspruch 1 beschrieben ist. Vorteilhafte Ausgestaltungen sind Gegenstand der Unteransprüche.

Um den o.g. Anforderungen gerecht zu werden liegt erfindungsgemäß das Verhältnis, q, des Außendurchmessers, $r_a$, zum Innendurchmesser, $r_i$, des Stentkörpers bei einem Wert von 1,2 und mehr.

Erfindungsgemäß wird hierbei das Verhältnis q in einem Bereich von $1,2 \leq q \leq 3,0$ gewählt, insbesondere von $1,2 < q \leq 2,8$, bevorzugt von $1,5 \leq q \leq 2,5$ und besonders bevorzugt von $1,8 \leq q \leq 2,2$.

Im Gegensatz hierzu liegt z. B. für Koronarstents der Wert q typischerweise in einem Bereich von kleiner 1,2.

Es hat sich jedoch gezeigt, dass bei der im Vergleich zu dem erfindungsgemäßen Stent für die Nasennebenhöhle geringeren Wanddicken des Koronarstents, die kontrollierte Freisetzung von Wirkstoff wie sie erfindungsgemäß gewünscht ist, nicht erzielt werden kann.

So kann der Quotient q hier als Berechnungsgrundlage dienen. Beispielsweise lässt sich für einen hohlen Zylinder das Volumen, V, aus der Höhe, h, dem Innendurchmesser, $r_i$, und q berechnen:

$$V = \pi \cdot h \cdot r_i^{\,2}\left(q^2 - 1\right) \qquad [1]$$

Gleichung 1 macht deutlich, dass das Volumen eines Koronarstents (mit $q=_{1,2}$) bei gleichem Innendurchmesser $r_i$ und gleicher Höhe h maximal ca. 15% des Volumens eines erfindungsgemäßen Nasennebenhöhlenplatzhalters (mit $q=2$) beträgt. Daraus resultiert, dass Stents mit $q=1,2$ oder kleiner maximal 1/7 der Wirkstoffdosis eines erfindungsgemäßen Nasennebenhöhlenplatzhalters aufnehmen können.

Der Quotient q hat auch gravierende Konsequenzen für die Freigabezeiträume, über die Wirkstoffe freigesetzt werden können. Für die Abschätzung der Freigabedauer, t, in Abhängigkeit von der Dicke eines Materials, I, und dem Diffusionskoeffizienten, D, wird in der Literatur der dimensionslose Ausdruck:

$$t = \frac{l^2}{D} \qquad [2]$$

verwendet (Cussler, E. L.; Diffusion: Mass Transfer in Fluid Systems, Cambridge Univ Press, 1996). Die Diffusionsstrecke in einem hohlen Zylinder lässt sich abschätzen als Hälfte der Differenz zwischen Außendurchmesser, $r_a$, und Innendurchmesser, $r_i$. Bei einem Stent mit konstantem Innendurchmesser, $r_i$, reduziert sich die Freigabedauer auf 4%, wenn q von 2 auf 1,2 gesenkt wird. Aus den oben genannten Gründen liegt für den beschriebenen Nasennebenhöhlenplatzhalter der Wert q vorzugsweise über 1,2, und insbesondere in einem Bereich $1,2 < q \leq 2,8$, besonders bevorzugt $1,5 \leq q \leq 2,5$ und insbesondere bevorzugt ist der Bereich $1,8 \leq q \leq 2,2$.

Gemäß einem weiteren Merkmal betrifft die Erfindung einen Platzhalter für die Nasennebenhöhle, bei dem die wirkstoffbeladene Schicht bzw. Schichten nach innen zu dem inneren Hohlraum hin durch eine Schicht abgetrennt sind, die aus einem Material besteht, das für den Wirkstoff undurchlässig oder zumindest nahezu undurchlässig ist. Dadurch werden Wirkstoffverluste vermieden und gleichzeitig wird die Zeitdauer, über die der Wirkstoff freigesetzt wird, erhöht.

Gravierende Unterschiede bestehen auch bzgl. der mechanischen Eigenschaften. Koronarstents, die über einen Katheter in die Blutgefäße eingebracht werden, müssen plastisch, d. h. irreversibel, verformbar sein. Durch ein irreversibles Aufweiten des Stents werden sie an der Gefäßwand fixiert.

Der im Rahmen dieser Erfindung entwickelte Platzhalter zum Einsatz in der Nasennebenhöhle zeichnet sich im Gegensatz dazu durch elastische Eigenschaften und damit reversible Verformbarkeit aus, u. a. aufgrund dieser Elastizität lässt sich der erfindungsgemäße Nasennebenhöhlenplatzhalter auf einfache Weise in den Öffnungen zur Nasennebenhöhle fixieren.

Mit dem erfindungsgemäßen wirkstoffbeladenen Platzhalter (Stent) ist es möglich, die Stirnhöhlenzugänge nicht nur über einen physikalisch/mechanischen-, sondern auch über einen pharmakologischen Mechanismus offen zu halten. Diese Platzhalter sind den operativ geschaffenen Zugängen zur Nasennebenhöhle angepasst und erfüllen zweierlei Funktion:

1. Sie halten die neu geschaffene 'Fensterung' der Stirnhöhle im Rahmen der minimal invasiven Ausräumung physikalisch offen. Dies wird zum einen durch das Anlegen des Platzhalters am operativ veränderten Gewebe erreicht und durch die Begünstigung des Sekretabflusses aus der Nebenhöhle unterstützt.

2. Die entwickelten Platzhalter können Wirkstoffe wie Arzneistoffe wie z.B. Kortikoide freigeben, die eine Gewebeneubildung bzw. die überschießende Wundheilung unterdrücken, und damit die operativ neu geschaffene Fensterung offen halten.

Um beide Funktionen in optimaler Weise erfüllen zu können, weist der "Stent" einige Designmerkmale auf, die nachfolgend anhand der Figuren näher beschrieben sind.
Der erfindungsgemäße Platzhalter ist ein Hohlkörper, der aus einer Hülle aufgebaut ist, die einen inneren Hohlraum umgibt und an zwei entgegengesetzten Enden jeweils eine Öffnung aufweist.
Vorzugsweise basiert der Hohlkörper auf einer Zylinderform, wobei er je nach Bedarf von der idealen Zylinderform mit insbesondere gleichmäßigem Durchmesser entlang des Schaftes abweichen kann.
So kann der Außendurchmesser entlang des Zylinderschaftes variieren, beispielsweise kann der Außendurchmesser in den Endbereichen nahe den Öffnungen größer als in dem mittleren Schaftbereich gewählt werden.
Der Außendurchmesser kann ausgehend von den Endbereichen in Richtung des mittleren Schaftbereich kontinuierlich abnehmen, in dem mittleren Bereich sanduhrartig verringert sein, wobei die spezielle Form des zylinderförmigen Grundkörpers je nach Bedarf beliebig angepasst werden kann.
Ebenso kann die Wanddicke des Zylinders variabel gewählt werden.

So zeigt:

Figur 1    eine erfindungsgemäß bevorzugte Ausgestaltung des Platzhalters in Zylinderform,

Figur 2    die Aufsicht auf einen Querschnitt durch einen erfindungsgemäßen wirkstoffbeladenen Platzhalter,

Figur 3    die Aufsicht auf einen Querschnitt auf eine weitere bevorzugte Ausgestaltung des erfindungsgemäßen Platzhalters als Reservoirsystem mit mehreren Schichten,

Figur 4    eine weitere Ausgestaltung des erfindungsgemäßen Platzhalters mit Perforationen in der Hülle,

Figur 5    eine Ausgestaltung des erfindungsgemäßen Platzhalters, wobei der Außendurchmesser des Zylinderschafts in den Endbereichen größer als zur Mitte hin ist, und zudem die Wanddicke zur Mitte des Zylinderschafts hin zunimmt,

Figur 6    eine weitere Ausgestaltung des erfindungsgemäßen Platzhalters als Matrixsystem mit mehreren Schichten, und

Figur 7    ein Diagramm mit dem Freisetzungsverlauf eines Wirkstoffs aus einem erfindungsgemäß bevorzugten Schichtmaterial.

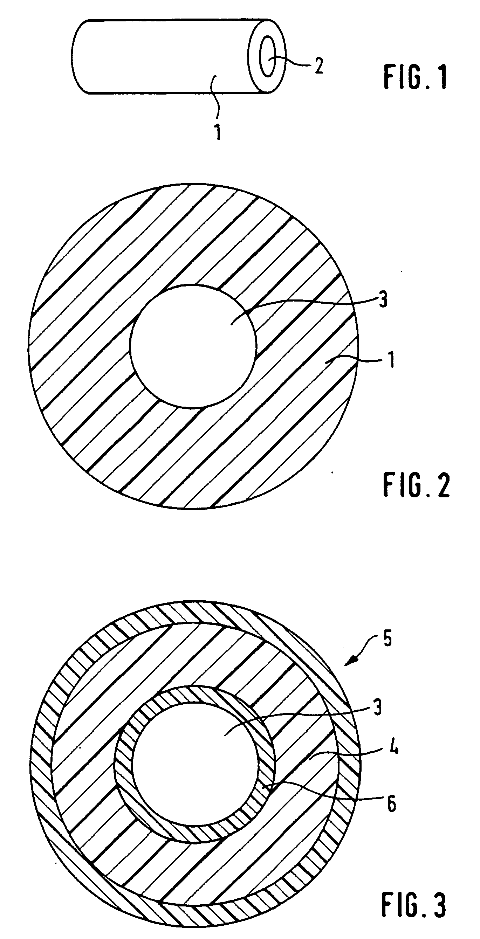

Die Geometrie des Platzhalters ist vorzugsweise die eines hohlen Zylinders, wie sie in Figur 1 zu sehen ist, wobei eine Hülle 1 einen inneren Hohlraum umgibt, der an den zwei entgegengesetzten Enden des Zylinderschafts Öffnungen 2 aufweist. Diese Zylinderform begünstigt aufgrund ihres röhrenartigen Designs den Sekretabfluss aus den Nebenhöhlen.

Die Länge des Zylinders wird hierbei vorzugsweise in einem Bereich von 5 bis 30 mm und sein Außendurchmesser in einem Bereich von 1 bis 30 mm gewählt.

Die Wandstärke ist in Abhängigkeit der physikalischen Eigenschaften der verwendeten Polymermaterialien sowie der eingesetzten Wirkstoffe und dem gewünschten Freisetzungsprofil innerhalb der vorstehend genannten Bereiche für das Verhältnis q von Außendurchmesser zu Innendurchmesser je nach Bedarf zu wählen.

Die mindestens zwei inneren Öffnungen 2 haben typischerweise einen Durchmesser in einem Bereich von 0,5 bis 25mm.

In Figur 2 ist ein Beispiel für einen Platzhalter mit monolitischem Aufbau gezeigt, wobei die Hülle 1 aus einer einzigen Schicht aufgebaut ist, und das Schichtmaterial die Matrix für den Wirkstoff bildet.

Figur 3 zeigt ein Beispiel für eine Ausgestaltung des Platzhalters als Reservoirsystem, wobei der Wirkstoff in einem Reservoir 4 enthalten ist. In diesem Fall liegt der Wirkstoff anders als bei dem Matrixsystem nicht in einem Matrixmaterial enthalten vor. Der Wirkstoff kann hierbei direkt die das Reservoir bildende Schicht ausbilden oder der Wirkstoff kann in einem entsprechenden Hohlraum vorgesehen sein. Der letztere Fall ist insbesondere für flüssige oder halbfeste Wirkstoffe oder für flüssige oder halbfeste Träger, die den Wirkstoff enthalten, geeignet. Der freizugebende Wirkstoff kann auch in dem Reservoirsystem aufgelöst oder suspendiert vorliegen.

Das den Hohlraum bildende freisetzungssteuernde Material ist von einer äußeren Membran 6 umgeben, die vorzugsweise aus einem Polymermaterial besteht, durch das der Wirkstoff hindurch diffundieren kann.

Vorzugsweise ist der das Reservoir 4 bildende Hohlraum nach innen zu dem inneren Hohlraum 3 durch eine Innenwand 6 abgetrennt, die vorzugsweise aus einem Material besteht, das für den Wirkstoff undurchlässig oder nahezu undurchlässig ist.

Eine derartige möglichst wirkstoffundurchlässige innere Schicht (Innenwand 6) eignet sich im Prinzip für alle wirkstofftragenden Systeme zur Vermeidung von Wirkstoffverlusten in Richtung des inneren Hohlraums 3.

Die Innenwand 6 kann hierbei aus einem entsprechenden Polymermaterial aber auch aus einem anorganischen Material wie einem Metall etc. bestehen.

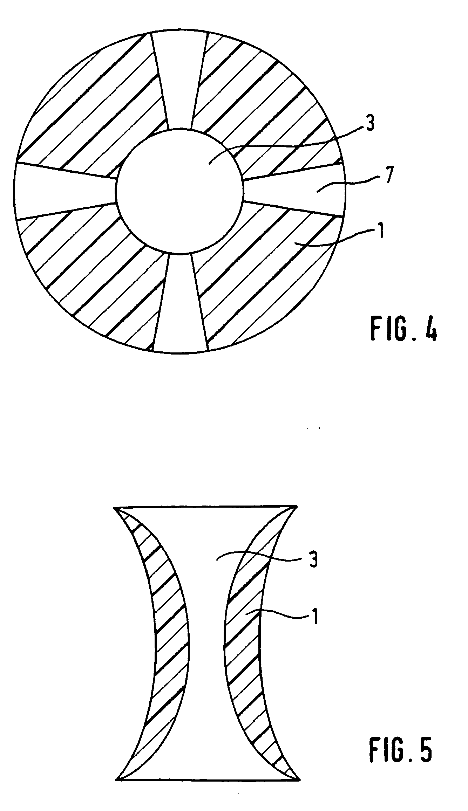

Die Hülle 1 kann Perforationen 7 aufweisen, - wie in Figur 4 gezeigt - die den inneren Hohlraum 3 mit der äußeren Oberfläche des Stents verbinden. Durch diese Maßnahme lässt sich ebenfalls der Sekretabfluss unterstützen.

Die Form und Anzahl der Perforationen 7 kann hierbei frei nach Bedarf gewählt werden.

Eine erfindungsgemäße Ausgestaltung mit variierendem Außendurchmesser ist in Figur 5 dargestellt. In der in Figur 5 gezeigten Ausführungsform ist der Außendurchmesser des Stents an den Endbereichen mit den Öffnungen 2 größer gewählt als in dem mittleren Bereich und nimmt kontinuierlich zur Mitte hin ab.

Wie hier gezeigt, kann zudem auch die Wandstärke variieren, wobei sie in diesem Fall zu den Endbereichen hin abnimmt.

Die Wanddicke, das heißt das Verhältnis q von Außendurchmesser zu Innendurchmesser, kann bei Bedarf in einzelnen Bereichen des Zylinderschafts, insbesondere in den Endbereichen oder aber auch in kurzen mittleren Abschnitten außerhalb des erfindungsgemäß einzustellenden Wertes für q liegen, solange die Funktionsfähigkeit des Stents nicht beeinträchtigt wird. So sollte zumindest in den Wundbereichen q innerhalb des vorstehend genannten erfindungsgemäßen Wertebereichs von 1,2 $\leq q \leq 3,0$ liegen. Für den Fall, dass in einzelnen Bereichen des Hohlkörpers die Wanddicke einen Wert q hat, der außerhalb des erfindungsgemäßen Wertebereichs liegt, sollten diese Bereiche oder dieser Bereich nicht mehr als 30 % des Hohlkörpers betragen.

Der erfindungsgemäße Stent kann aus einer oder mehreren Schichten gebildet sein, wobei die Schichten aus dem gleichen und/oder unterschiedlichen Polymeren bestehen können. Einzelne Bereiche einer Schicht, z.B. die Endbereiche nahe den Öffnungen 2, können aus einem Material gefertigt sein, das von dem Material für die verbleibenden Schichtbereiche unterschiedlich ist. Mit anderen Worten, eine Schicht kann mindestens einen Bereich enthalten, der aus einem anderen Material als die übrige Schicht gebildet ist.

Zudem kann der erfindungsgemäße Stent neben wirkstoffbeladenen Schichten wirkstofffreie Schichten aufweisen.

Bei Bedarf kann der erfindungsgemäße Stent von einem geeigneten äußeren Überzug umgeben sein.

Im Gegensatz zu Koronarstents liegt der erfindungsgemäße Platzhalter nicht notwendiger Weise homogen und plan auf dem Gewebe auf. Dieser Umstand verlangt eine besondere Konstruktion, damit sich z.B. nicht Sekret zwischen dem Platzhalter und der Nebenhöhlenwand langfristig stauen kann. Die Sekretdrainage kann durch Perforationen 7 in der Wand des Platzhalters erleichtert werden (siehe Querschnitt dargestellt in Figur 4).

In seiner Form kann der Platzhalters eine "sanduhrförmige" Übergangszone von Stirnhöhle zum Naseninneren abbilden (Figur 5) und gestattet durch eine zentrale Öffnung die Endoskopie der Stirnhöhle.

Gemäß einer weiteren Ausgestaltung kann der Platzhalter auf seiner äußeren Oberfläche Unebenheiten wie Buckel etc. aufweisen. In diesem Fall besteht der Kontakt mit der Nasenwand über diese Unebenheiten, wobei einerseits die Kontaktfläche verringert werden kann und dennoch ein ausreichend fester Halt sichergestellt ist. Die entstehenden Hohlräume zwischen äußerer Oberfläche des Stents und Nasenwand fördern gleichzeitig den Sekretabfluss.

Der Platzhalter ist darüber hinaus vorzugsweise so geschaffen, dass ein Absaugen der Nasennebenhöhle durch den Platzhalter hindurch möglich bleibt. Dies wird durch eine relativ geringe Länge ermöglicht. Der Platzhalter ist deshalb vorzugsweise so konstruiert, dass er unmittelbar vor der Applikation auf die gewünschte Länge zugeschnitten werden kann.

Der Platzhalter muss entgegen der Schwerkraft im Stirnhöhleneingang "verankert" werden. Diese Verankerung kann durch eine "Ballonierung" des Implantates d.h. eine Aufweitung des Platzhalterendes in der Stirnhöhle oder eine Fixierung mittels einer Naht an der Nasenscheidewand erzielt werden. Darüber hinaus kann der Stent aus Materialien bestehen, die eine Verankerung und die Formanpassung begünstigen. In diesem Zusammenhang lassen sich ,shape memory polymere' (z. B. US 5,139,832, US 5,189,110) oder quellende Polymere einsetzen (z. B. DE 4 032 096).

Während erstere ihre Gestalt bei Körpertemperatur ändern, kommt es bei quellenden Substanzen zu einer Volumenzunahme des Materials durch Wasseraufnahme und damit zu einer Erhöhung des Stentdurchmessers nach dessen Applikation. Die Materialien passen sich dabei dem Defekt optimal an und verhindern dadurch ein Verrutschen des Stents. Durch ihre gute Wasserdurchlässigkeit verhindern quellende Polymere einen Sekretstau an der Kontaktfläche zum Gewebe.

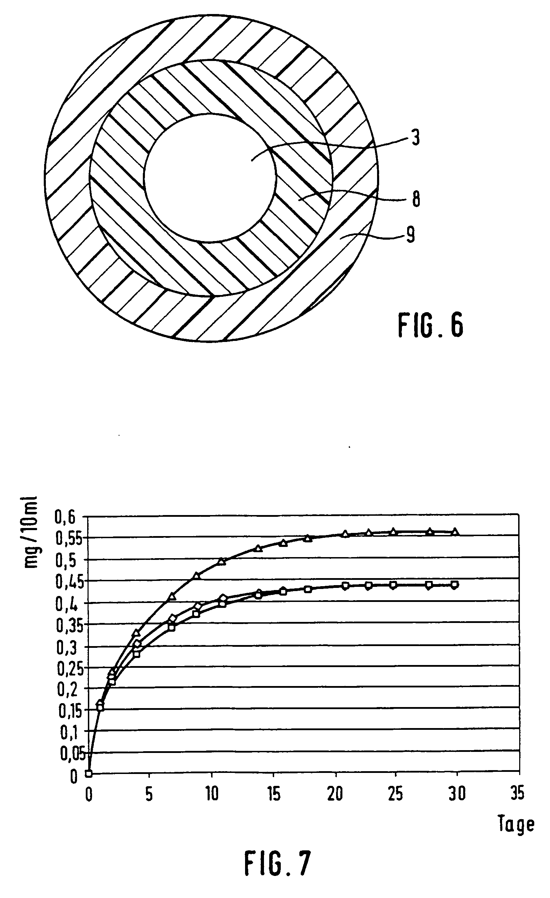

Ein Beispiel hierfür ist in Figur 6 gezeigt, wobei die äußere Schicht 9 aus einem verformbaren Polymer besteht und eine wirkstoffbeladene Polymerschicht 8 umgibt.

Der Platzhalter in der Nase ist im Gegensatz zu Platzhaltern in Gefäßen einem bakterienbeladenen Milieu (Schleimhautwunde mit freiem Kontakt zur Außenluft) ausgesetzt. Durch eine entsprechende Formgebung wird eine Verkrustung und bakterielle Kontamination verzögert. Dies kann zum Beispiel durch einen ausreichend großen Innendurchmesser des Platzhalters erreicht werden, der den Sekretabfluss fördert. Die verwendeten Materialien können darüber hinaus an der Oberfläche so modifiziert sein, dass der Sekretabfluss begünstigt und eine bakterielle Kontamination vermieden wird. Ein Beispiel ist die Hydrophilisierung der Oberflächen. Dazu kann die Innenseite des Hohlkörpers mit einer Polymerschicht ausgekleidet sein, die gut benetzbar ist und vorzugsweise Wasserkontaktwinkel < 45° aufweist.

Alternativ dazu lassen sich Polymere einsetzen, deren Oberfläche chemisch modifiziert wurden, wie zum Beispiel durch die chemische Bindung hydrophiler Substanzen oder durch die Behandlung mit Gasplasma.

Zur Vermeidung einer bakteriellen Kontamination kann der Platzhalter zusätzlich mit bakterizid- oder bakteriostatisch wirksamen Substanzen beladen sein.

Um die vielfältigen Funktionen des Stent zu gewährleisten kann vor allem die Ausführung des Matrixsystems aus mehreren Polymerschichten bestehen wie der Querschnitt in Figur 6 beispielhaft zeigt. Die Anzahl der Schichten ist nicht, wie in der Abbildung gezeigt, auf 2 beschränkt. So können mehrere Schichten, die unterschiedliche Funktionen erfüllen miteinander kombiniert werden. Einzelne Schichten können arzneistofffrei, bzw. mit einem -, oder mit mehreren Arzneistoffen beladen sein. Bei Beladung unterschiedlicher Schichten mit verschiedenen Arzneistoffen lassen sich diese aus dem Platzhalter mit unterschiedlicher Kinetik freisetzen. Die Dicke einzelner Polymerschichten kann dabei beliebig dünn sein z.B. im Bereich weniger Mikrometer.

Der Platzhalter kann vor der Applikation bereits vorgeformt sein, oder aber auch durch Verarbeitung einer Vorstufe zu seiner endgültigen Geometrie geformt werden. Verfahren, wie zum Beispiel die Extrusion oder Spritzgussverfahren eignen sich hervorragend zur Herstellung vorgeformter Platzhalter. Für die Herstellung aus Vorstufen können zum Beispiel Polymerfilme zu Hohlkörpern gerollt und über eine Naht fixiert werden.

Die Materialien, aus denen der Platzhalter hergestellt werden kann, können bioabbaubare oder auch nicht bioabbaubare Materialien sein sowie Kombination hiervon.

Als bioabbaubare Materialien kommen zum Beispiel die Polymere der Milchsäure oder Glykolsäure sowie deren Copolymere in Betracht. Weitere geeignete Beispiele finden sich in der Literatur (K. Park, W.S.W Shalaby, H. Park, Biodegradable Hydrogels for Drug Delivery, Technomic Publishing Inc. Lancaster 1993; A.

Domb, J. Kost, D.M. Wiseman, Handbook of Biodegradable Polymers, Harwood Academic Publishers, 1997).

Während bioabbaubare Materialien den Vorzug besitzen, nach der Anwendung nicht entfernt werden zu müssen, lassen sich nicht bioabbaubare Materialien im Einsatzgebiet des Platzhalters besser fixieren. Beispiele für solche Materialien sind Silikone, Polyacrylate und Polymethacrylate sowie deren Copolymere (Eudragit®), Poly(ethylenvinylacetat)-Copolymer und andere Verbindungen, wie sie in der Polymerliteratur beschrieben und für medizinische Anwendungen bekannt sind.

Die Polymere sollen vorzugsweise flexibel sein, damit sie sich dem Wundgebiet anpassen. Sie sollen darüber hinaus elastisch genug sein, um in der Fensterung zu verbleiben und sie sollen biokompatibel sein, d.h. gute Verträglichkeit gegenüber Zellen und Geweben aufweisen. Um die mechanische Verhaftung des Platzhalters an der Fensterung zu gewährleisten, können die erwähnten Polymere mit anderen Materialien kombiniert werden, so z.B. mit Metallen, um einen sicheren Sitz des "Stents" bei geringeren Wanddicken zu gewährleisten. Diese Metalle können in die Wand des Zylinders eingearbeitet sein.

Die Polymere können zu den in Figur 1 und 2 gezeigten Platzhaltern durch verschiedene technische Verfahren verarbeitet werden, so z.B. durch Extrusion oder Spritzgussverfahren oder durch Polymerisation in geeigneten Formen.

Ein einfaches Verfahren zur Herstellung stellt das Ausgießen von Polymerlösungen dar (solvent casting). Dazu werden die Polymere in organischem Lösungsmittel gelöst, und die Lösung auf eine inerte Oberfläche ausgegossen oder aufgesprüht. Nach dem Verdampfen des Lösungsmittels erhält man trockene, wirkstoffbeladene Polymerfilme, die sich in beliebige, zum Beispiel rechteckige Formen zuschneiden lassen.

Während durch Extrusion oder Spritzguss unmittelbar Schläuche erhalten werden, lassen sich aus rechteckigen Polymerfilmen unmittelbar vor Einsetzen in den Patienten individuell angepasste Röhrchen formen. Dies kann durch mehrfaches Rollen des Polymerfilms geschehen oder durch mechanische Verhaftung oder Verkleben gegenüberliegender Filmkanten.

Durch die Art der Herstellung lassen sich die Polymereigenschaften so steuern, dass entweder glatte oder poröse Oberflächen entstehen. Dies hat Einfluss auf die Geschwindigkeit der Wirkstofffreigabe und ggf. die Wechselwirkungen zwischen Platzhalter und Wundrändern.

Die Oberflächen des Platzhalters zur Gewebe- und zur Sekretseite hin können darüber hinaus so verändert werden, dass sie den Anforderungen an ihre Funktionen optimal gerecht werden. Die Innenseite des Zylinders zum Hohlraum des Platzhalters kann z. B. an ihrer Oberfläche physikochemisch so verändert werden, dass es zu einer besseren Benetzung mit Sekret und damit zu einer verbesserten Sekretdrainage kommen kann. Beispiele sind die o.g. Hydrophilisierung von Oberflächen oder die kovalente Bindung hydrophiler Substanzen an die Polymeroberflächen.

Die Oberfläche zur Gewebeseite kann chemisch so veränderte werden, dass die Gewebeverträglichkeit verbessert wird. Dies lässt sich durch einen Überzug mit Materialien in Form dünner Filme oder durch Anbindung oder Auftragung funktioneller Gruppen oder ganzer Moleküle, die mit dem biologischen System eine Wechselwirkung eingehen, erreichen. So führt die Verankerung von Polyethylenglykolketten an der Oberfläche zu einer verringerten Zellanheftung, was das Entfernen des Platzhalters erleichtert und seine Verträglichkeit mit dem Wundgewebe steigert.

Die Wirkstoffe können je nach Bedarf, Verwendungszweck, gewünschter Eigenschaft etc. gewählt werden. Sie können auch in Kombination eingesetzt werden. Insbesondere werden die erfindungsgemäßen Stents mit Arzneistoffen beladen.

Als Arzneistoffe kommen üblicher Weise Substanzen zur Anwendung, die Einfluss auf das Verhalten von Zellen und Geweben nehmen können. Insbesondere sollen sie ein unkontrolliertes Gewebewachstum verhindern. Zu diesem Zweck eignen sich Vertreter aus der Gruppe der Glucokortikoide wie z.B. Cortisol, Cortison, Prednison, Prednisolon, 6-Methylprednisolon, Dexamethason, Flutrokortison, Desoxikortonacetat. Weitere Beispiele sind Proteine aus dem Bereich der Cytokine und Wachstumsfaktoren von denen ebenfalls einigen zellwachstumshemmende Eigenschaften zugesprochen werden. Darüberhinaus eignen sich Tyrosinkinaseinhibitoren, Antisense Oligonukleotide und Mitosehemmstoffe wie Mitomycin dazu, den proliferativen Einfluss von Wachstumsfaktoren im Rahmen der Wundheilung auszuschalten.

Die Wirkstoffe können über einen längeren Zeitraum aus dem Platzhalter freigesetzt werden. Je nach Design und verwendetem Material sind Freigaben bis zu mehreren Jahren realisierbar. Vorzugsweise erstreckt sich die Freigabe über einen Zeitraum von 2 bis 12 Wochen. Als freigabesteuernde Prinzipien kommen dabei, neben der Wandstärke ausgedrückt als Verhältnis q, vor allem die Diffusion und die Polymerquellung für nicht bioabbaubare Polymere in Frage. Bei Verwendung von bioabbaubaren Materialien, d. h. solchen, die sich im Rahmen der Anwendung auflösen, spielt auch die Polymererosion eine bedeutende Rolle (Göpferich, Polymer Degradation and Erosion: Mechanismus and Applications, Eur. J. Pharm. Biopharm., 42 (1996) 1-11).

Wird der Platzhalter aus den bevorzugten nicht abbaubaren Materialien hergestellt, wird der Wirkstoff vorzugsweise aus einem Reservoir- oder einem Matrixsystem freigesetzt. In beiden Fällen wird der Wirkstoff hierbei über Diffusion freigesetzt. Die Wirkstofffreigabe kann über mehrere Faktoren beeinflusst werden. Durch Verändern der Geometrie kann der Wirkstoff über verschieden lange Zeiten freigesetzt werden. Darüber hinaus ist es möglich, über den Beladungsgrad die Kinetik der Wirkstofffreigabe zu kontrollieren.

Die Beladung, insbesondere bei der Ausführung als Matrixsystem, liegt vorzugsweise in einem Bereich bis zu 30 Gew. % bezogen auf das Gesamtsystem. Die minimale Beladung hängt u. a. von der Potenz des Wirkstoffs und von der erwünschten Freigabedauer ab.

Zur weiteren Beeinflussung der Diffusion können der Polymermatrix beziehungsweise den Polymeren Zusätze zugesetzt werden. So führen inerte anorganische Materialien, wie z. B. Siliziumdioxid, zu einer Verringerung der Freigabegeschwindigkeit. Je nach Polymertyp kann durch Weichmacherzusätze die Freigabegeschwindigkeit gesteigert werden. Im Rahmen der Polymerquellung lässt sich die Quellung durch osmotische Zusätze in das Polymer steigern, dadurch kann je nach Wirkstoffeigenschaften, die Freigabegeschwindigkeit erhöht werden.

Um über Erosion die Wirkstofffreigabe zu kontrollieren, kann der Typ des bioabbaubaren Polymers entsprechend der Anwendung zugeschnitten werden. So ist z.B. bei Poly (D,L-lactid-co-glykolid) bekannt, dass sich über die Steigerung des Glykolidanteils die Freigabegeschwindigkeit und auch die Erosionsgeschwindigkeit steuern lässt.

Gegenstand der Erfindung ist ein Platzhalter (Stent), der nach operativer Öffnung der Nasennebenhöhle (vornehmlich der Stirnhöhle) in die geschaffene Fensterung zur Nase eingesetzt wird. Der neu entwickelte Platzhalter verhindert eine post-operative narbige Verengung indem er zwei konventionelle Behandlungsansätze für den operativ neu geschaffenen Stirnhöhlenzugang in sich vereinigt:

1. Der Platzhalter fungiert als physikalische Barriere, die den Zugang zur Stirnhöhle mechanisch offen hält.

2. Der Platzhalter setzt Arzneistoffe frei, die das Wachstum des Gewebes um den neu geschaffenen Zugang zur Stirnhöhle kontrollieren.

Das Material des Platzhalters besitzt vorzugsweise die mechanische Eigenschaften eines Elastomers wie z.B. Silikon, ein in der HNO-Chirurgie bewährter Stoff für Platzhalter. Aufgrund der bevorzugten Geometrie, die im wesentlichen der eines hohlen Zylinder entspricht, kann Sekret aus den Nebenhöhlen abfließen. Darüber hinaus wirkt das Material als lokales Freigabesystem für Arzneistoffe wie z.B. Kortikoide. Durch Form und Funktion sorgt der Stent für einen festen Sitz und erlaubt gleichzeitig die optimale Sekretdrainage. Die kontinuierliche Freigabe einer definierten Menge von Arzneistoff wird vorzugsweise über einen Zeitraum von 8 Wochen gewährleistet. Die vorgesehene Liegedauer des Implantates beträgt vorzugsweise ebenfalls 8 Wochen. Zur Herstellung lassen sich z.B. arzneistoffbeladene Folien zu einem Zylinder rollen und mit einer chirurgischen Naht stabilisieren. Der Platzhalter wird intra operationem in den neu geschaffenen Stirnhöhlenzugang eingeführt.

Bei Bedarf wird er durch seine besondere Form, die verwendeten Materialien, seinen Aufbau und/oder mit einer chirurgischen Naht im Operationsgebiet fixiert, um einer Verlagerung vorzubeugen. Neben der Anwendung in Fenstern zur Nasennebenhöhle ist ein Einsatz im Mittelohr und der Trachea möglich.

**1. Beispiel: Herstellung eines Dexamethasonbeladenen Polymerfilms**

Der Film weist folgende Zusammensetzung auf:

| | |
|---|---|
| Evatane 40-55 (mit Aceton gereinigt) | 17,955g |
| Dexamethason DAB 10/Ph.Eur. | 0,045g |
| Dichlormethan p.A | 98 ml |
| Aceton p.A | 4,5ml |

Zunächst wird das verwendete Polymer, ein Poly(ethylen-vinylacetat)-copolymer von Additiven befreit, die im Rahmen der Herstellung von Evatane 40-55 zugesetzt werden. Dazu wird 50g Evatane 40-55 in einen 500ml Jodzahlkolben mit Magnetrührstab eingewogen. 250ml Aceton p.a. werden mit einem Messzylinder abgemessen und zum Polymer gegeben. Der Ansatz wird auf dem Magnetrührer ca. 1 Woche gerührt und anschließend das Aceton dekantiert. Das Polymer wird dreimal mit 80 ml Aceton p.a. gewaschen und die Waschflüssigkeit verworfen. Das Extraktions- und Waschprotokoll wird einmal mit Aceton und zweimal mit Ethanol unter Verwendung derselben Volumina wiederholt. Anschließend wird das Polymer in einer Kristallisierschale in einer laminar airflow box 48h und anschließend im Exsiccator unter Vakuum getrocknet.

In einen 250ml Jodzahlkolben werden 17,955g Evatane 40-55 eingewogen. Dazu werden das Dichlormethan gegeben und auf dem Magnetrührer über 12h gerührt. Das Dexamethason wird im Aceton gelöst und zur Polymerlösung gegeben. Anschließend lässt man den Ansatz 10min. ohne rühren stehen, um Luftblasen zu entfernen. Die Lösung wird in eine plane Teflonform mit einer Fläche von 15cm$^2$ gegossen und in einer laminar airflow box über. 4 Tage getrocknet.

Der getrocknete Film wird aus der Teflonform gezogen und in Stücke beliebiger Größe geschnitten. Die Filmdicke beträgt ca. 0.8 - 1 mm. Die Polymerfilme werden zu einem hohlen Zylinder aufgerollt und an den Berührungsstellen vorzugsweise durch eine Naht mit einem biokompatiblen Nahtmaterial so fixiert, dass sich der Zylinder aufgrund der Elastizität des Materials nicht aufrollt. Das so geformte Röhrchen wird dann in die Fensterung zur Nasennebenhöhle eingesetzt.

**2. Beispiel: Freigabe von Dexamethason aus dem Polymerfilm in Beispiel 1**

Aus dem in Beispiel 1 beschriebenen Film wurden runde Stücke mit einem Durchmesser von 1,2 cm Durchmesser geschnitten und die Freigabe in vitro bestimmt. Dazu wurden die mit 0,25% Dexamethason beladenen Polymerplättchen in verschließbaren Glasgefäßen in 10ml Phosphatpuffer bei 37°C gelagert. Dem Ansatz wurden in regelmäßigen Abständen Proben entnommen und durch frischen Puffer ersetzt. Der Dexamethasongehalt wurde per HPLC bestimmt. Figur 7 zeigt die Freigabe von Dexamethason über die Zeit.

**Bezugszeichenliste**

1. Hülle
2. Öffnung
3. innerer Hohlraum
4. Reservoirbereich
5. Membran
6. wirkstoffundurchlässige Schicht
7. Perforation
8. wirkstoffbeladene Polymerschicht
9. verformbare Polymerschicht

**Patentansprüche**

1. Platzhalter zur Implantation in einer Öffnung oder Fensterung einer Nebenhöhle, dem Mittelohr oder der Trachea, wobei der Platzhalter eine Hülle (1) umfasst, die einen Hohlkörper bildet und einen inneren Hohlraum (3) umgibt, wobei der Hohlkörper einen mittleren Bereich und zwei einander entgegengesetzte Enden mit zwei Öffnungen (2) aufweist, **dadurch gekennzeichnet, dass** die Hülle (1) ferner mindestens eine stoffhaltige Schicht umfasst, die eine mit einem Wirkstoff beladene Matrix umfasst, wobei der Wirkstoff wenigstens einen Stoff umfasst, ausgewählt aus der Gruppe bestehend aus Kortikoiden, Proteinen aus dem Bereich der Cytokine und Wachstumsfaktoren, die zellwachstumshemmende Eigenschaften haben, Tyrosinkinaseinhibitoren, Antisense-Oligonukleotide und Mitosehemmstoffe, die unkontrolliertes Gewebewachstum verhüten, und wobei der Hohlkörper des Platzhalters aus einem biokompatiblen Polymer gebildet ist, das flexibel ist, um sich an die Fensterung anzupassen, und elastisch genug ist, um in der Fensterung zu bleiben, so dass der Platzhalter elastische Eigenschaften aufweist und reversibel verformbar ist, so dass der Platzhalter auf einfache Welse in der genannten Öffnung fixiert werden kann.

2. Platzhalter nach Anspruch 1, wobei das Verhältnis von Außendurchmesser $r_a$ zu Innendurchmesser $r_i$ des Hohlkörpers größer oder gleich 1,2 und kleiner oder gleich 3,0 ist.

3. Platzhalter nach Anspruch 1 oder 2, **dadurch ge-** **kennzeichnet**, das der innere Hohlraum (3) von der mindestens einen stoffhaltigen Schicht, die die mit dem Wirkstoff beladene Matrix umfasst, durch eine innere Schicht (6) getrennt ist, die für den Wirkstoff im Wesentlichen undurchlässig ist.

4. Platzhalter nach einem der Ansprüche 1 bis 3, **dadurch gekennzeichnet, dass** der Hohlkörper vor der Implantation zylinderförmig ist.

5. Platzhalter nach einem der vorhergehenden Ansprüche, **dadurch gekennzeichnet, dass** der zylinderförmige Hohlkörper eine variierende Wandstärke hat.

6. Platzhalter nach einem der vorhergehenden Ansprüche, **dadurch gekennzeichnet, dass** die Hülle (1) des Hohlkörpers eine oder mehrere Perforationen (7) aufweist, die das Innere (3) des Hohlkörpers mit der Außenfläche verbinden.

7. Platzhalter nach einem der vorhergehenden Ansprüche, **dadurch gekennzeichnet, dass** der Hohlkörper ein Matrixsystem ist, das aus zwei oder mehr Schichten aufgebaut ist.

8. Platzhalter nach Anspruch 7, **dadurch gekennzeichnet, dass** das Matrixsystem eine oder mehrere wirkstofffreie Schichten aufweist.

9. Platzhalter nach Anspruch 7 oder 8, **dadurch gekennzeichnet, dass** das Matrixsystem zwei oder mehrere Schichten aufweist, die mit einem Wirkstoff beladen sind, wobei der Wirkstoff gleich oder verschieden ist.

10. Platzhalter nach einem der Ansprüche 1 bis 6, **dadurch gekennzeichnet, dass** der Hohlkörper ein Reservoirsystem bildet, wobei der Wirkstoff in einem Bereich (4) des Hohlkörpers vorliegt, der durch eine Membran (5) nach außen hin abgetrennt ist.

11. Platzhalter nach einem der vorhergehenden Ansprüche, **dadurch gekennzeichnet, dass** zumindest die äußere Schicht des Hohlkörpers aus einem Material gefertigt ist, das ausgewählt ist unter einem memory-shape-Polymer oder einem quellenden Material.

12. Platzhalter nach einem der vorhergehenden Ansprüche, **dadurch gekennzeichnet, dass** der Platzhalter aus einem bioabbaubaren und/oder nicht bioabbaubaren Polymer besteht.

13. Platzhalter nach Anspruch 12, **dadurch gekennzeichnet, dass** die Schichten aus gleichen und/oder verschiedenen Polymermaterialien bestehen, wobei die Polymermaterialien aus bioabbaubaren und/oder nicht bioabbaubaren Polymeren ausgewählt sind.

**14.** Platzhalter nach einem der vorhergehenden Ansprüche, **dadurch gekennzeichnet, dass** der Platzhalter oder einzelne Schichten des Platzhalters aus mindestens einem Polymer bestehen, das ausgewählt ist unter Poly(alphahydroxyestern), Polyacrylaten, Ethylenvinylacetat-Copolymer oder Silikon.

**15.** Platzhalter nach einem der vorhergehenden Ansprüche, **dadurch gekennzeichnet, dass** der Wirkstoff in dem Platzhalter oder in einer Schicht des Platzhalters in einem Gehalt von bis zu 30 Gew.-% vorliegt.

**16.** Platzhalter nach einem der vorhergehenden Ansprüche, **dadurch gekennzeichnet, dass** die äußere Oberfläche des Platzhalters Unebenheiten aufweist.

**17.** Platzhalter nach einem der vorhergehenden Ansprüche, **dadurch gekennzeichnet, dass** der Platzhalter zusätzliche Bereiche aufweist, in denen das Verhältnis von Außendurchmesser zu Innendurchmesser q kleiner als 1,2 oder größer als 3 ist.

**18.** Platzhalter nach einem der vorhergehenden Ansprüche, **dadurch gekennzeichnet, dass** das Kortikoid ein Glucocorticoid umfasst, ausgewählt aus der Gruppe bestehend aus Cortisol, Cortison, Prednison, Prednisolon, 6-Methylprednisolon, Dexamethason, Fludrocortison und Desoxycortonacetat.

**19.** Platzhalter nach einem der vorhergehenden Ansprüche, wobei mindestens ein Ende des Hohlkörpers nach dem Einsetzen in der Nebenhöhle ausdehnbar ist.

**20.** Platzhalter nach einem der vorhergehenden Ansprüche, **dadurch gekennzeichnet, dass** der Platzhalter aus bioabbaubarem Material besteht, ausgewählt aus der Gruppe der Polymere und der Copolymere der Milchsäure und der Polymere und der Copolymere der Glykolsäure.

**21.** Platzhalter nach einem der vorhergehenden Ansprüche, **dadurch gekennzeichnet, dass** der Platzhalter aus nicht bioabbaubarem Material besteht, ausgewählt aus der Gruppe bestehend aus Silikonen, Polyacrylaten, Polymethacrylaten, Copolymern von Polyacrylaten und Polymethacrylaten und Poly(ethylenvinylacetat)-Copolymeren.

**Claims**

**1.** A spacing device for implantation in an opening or fenestration of a paranasal sinus, the middle ear or the trachea, the spacing device comprising a sheath (1) forming a hollow body and surrounding an inner cavity (3), with the hollow body comprising a central area and two opposite ends with two openings (2), **characterised in that** the sheath (1) also comprises at least one substance-containing layer comprising a matrix charged with an active ingredient, wherein the active ingredient includes at least one substance, selected from the group consisting of corticoids, proteins from the group of cytokines and growth factors, having cell growth inhibiting properties, tyrosine kinase inhibitors, antisense oligonucleotides and mitotic inhibitors, which prevent uncontrolled tissue growth, and wherein the hollow cavity of the spacing device is made from a biocompatible polymer that is flexible in order to adapt to the fenestration, and is sufficiently elastic to remain in the fenestration, so that the spacing device comprises elastic properties and is reversibly deformable, so that the spacing device can be fixed in a simple manner in the said opening.

**2.** A spacing device according to Claim 1, wherein the ratio of the external diameter $r_a$ to the internal diameter $r_i$ of the hollow body is greater than or equal to 1.2 and less than or equal to 3.0.

**3.** A spacing device according to Claim 1 or 2, **characterised in that** the inner cavity (3) of the at least one substance-containing layer, which comprises the matrix charged with the active ingredient, is separated by an inner layer (6) that is substantially impermeable to the active ingredient.

**4.** A spacing device according to one of Claims 1 to 3, **characterised in that** the hollow body is cylindrical prior to implantation.

**5.** A spacing device according to one of the preceding claims, **characterised in that** the cylindrical hollow body has a varying wall thickness.

**6.** A spacing device according to one of the preceding claims, **characterised in that** the sheath (1) of the hollow body comprises one or more perforations (7) connecting the interior (3) of the hollow body to the external surface.

**7.** A spacing device according to one of the preceding claims, **characterised in that** the hollow body is a matrix system composed of two or more layers.

**8.** A spacing device according to Claim 7, **characterised in that** the matrix system comprises one or more layers devoid of active ingredient.

**9.** A spacing device according to Claim 7 or 8, **characterised in that** the matrix system comprises two or more layers charged with an active ingredient, the active ingredient being the same or different.

**10.** A spacing device according to one of Claims 1 to 6, **characterised in that** the hollow body forms a reservoir system, the active ingredient being present in a region (4) of the hollow body that is outwardly separated by a membrane (5).

**11.** A spacing device according to one of the preceding claims, **characterised in that** at least the outermost layer of the hollow body is manufactured from a material which is selected from a memory shape polymer or a swelling material.

**12.** A spacing device according to one of the preceding claims, **characterised in that** the spacing device is made from a biodegradable and/or non-biodegradable polymer.

**13.** A spacing device according to Claim 12, **characterised in that** the spacing device is made of identical and/or different polymer materials, with the polymer materials being selected from biodegradable and/or non-biodegradable polymers.

**14.** A spacing device according to one of the preceding Claims, **characterised in that** the spacing device or individual layers of the spacing device are made of at least one polymer which is selected from poly(alpha-hydroxy esters), polyacrylates, ethylene vinyl acetate copolymer or silicone.

**15.** A spacing device according to one of the preceding claims, **characterised in that** the active ingredient in the spacing device or in a layer of the spacing device is present in a concentration of up to 30% by weight.

**16.** A spacing device according to one of the preceding Claims, **characterised in that** the outer surface of the spacing device comprises uneven areas.

**17.** A spacing device according to one of the preceding Claims, **characterised in that** the spacing device comprises additional areas in which the ratio of the external diameter to the internal diameter q is less than 1.2 or greater than 3.

**18.** A spacing device according to one of the preceding Claims, **characterised in that** the corticoid includes a glucocorticoid, selected from the group consisting of cortisol, cortisone, prednisone, prednisolone, 6-methylprednisolone, dexamethason, fludrocortisone and deoxycortone acetate.

**19.** A spacing device according to one of the preceding Claims, **wherein** at least one end of the hollow body is expandable after insertion in the paranasal sinus.

**20.** A spacing device according to one of the preceding Claims, **characterised in that** the spacing device is made from biodegradable material, selected from the group of polymers and copolymers of lactic acid and the polymers and copolymers of glycolic acid.

**21.** A spacing device according to one of the preceding Claims, **characterised in that** the spacing device is made from non-biodegradable material, selected from the group consisting of silicones, polyacrylates, polymethyacrylates, copolymers of polyacrylates and polymethyacrylates and poly(ethylenevinylacetate) copolymers.

**Revendications**

**1.** Implant à implanter dans une ouverture ou une fenestration d'un sinus, dans l'oreille moyenne ou la trachée, ledit implant comprenant une gaine (1) qui forme un corps creux et enveloppe une cavité intérieure (3), ledit corps creux comportant une zone centrale et deux extrémités opposées l'une à l'autre avec deux ouvertures (2), **caractérisé en ce que** la gaine (1) comprend en outre au moins une couche contenant une substance et comprenant une matrice chargée d'un agent actif, ledit agent actif comprenant au moins une substance sélectionnée dans le groupe composé des corticoïdes, des protéines de l'ordre des cytokines et des facteurs de croissance pourvus de propriétés inhibitrices de la croissance cellulaire, des inhibiteurs des tyrosine-kinases, des oligonucléotides anti-sens et des agents anti-mitotiques prévenant une croissance tissulaire incontrôlée, et ledit corps creux de l'implant étant formé d'un polymère bio-compatible, flexible pour pouvoir s'ajuster à la fenestration, et suffisamment élastique pour demeurer dans la fenestration, de telle manière que l'implant présente des propriétés élastiques et soit déformable de manière réversible de manière à pouvoir être aisément fixé dans ladite ouverture.

**2.** Implant selon la revendication 1, où le rapport entre le diamètre extérieur $r_a$ et le diamètre intérieur $r_i$ du corps creux est supérieur ou égal à 1,2 et inférieur ou égal à 3,0.

3. Implant selon la revendication 1 ou la revendication 2, **caractérisé en ce que** la cavité intérieure (3) est séparée par une couche intérieure (6), essentiellement imperméable à l'agent actif, de la ou des couches contenant une substance et comprenant la matrice chargée de l'agent actif.

4. Implant selon l'une des revendications 1 à 3, **caractérisé en ce que** le corps creux est de forme cylindrique avant l'implantation.

5. Implant selon l'une des revendications précédentes, **caractérisé en ce que** le corps creux cylindrique présente une épaisseur de paroi variable.

6. Implant selon l'une des revendications précédentes, **caractérisé en ce que** la gaine (1) du corps creux présente une ou plusieurs perforations (7) qui relient l'intérieur (3) du corps creux à la surface extérieure.

7. Implant selon l'une des revendications précédentes, **caractérisé en ce que** le corps creux est un système matriciel structuré en deux ou en plusieurs couches.

8. Implant selon la revendication 7, **caractérisé en ce que** le système matriciel comporte une ou plusieurs couches exemptes d'agent actif.

9. Implant selon la revendication 7 ou la revendication 8, **caractérisé en ce que** le système matriciel comporte deux ou plusieurs couches chargées d'un agent actif, l'agent actif étant identique ou différent.

10. Implant selon l'une des revendications 1 à 6, **caractérisé en ce que** le corps creux forme un système de réservoir, l'agent actif étant présenté dans une région (4) du corps creux séparée de l'extérieur par une membrane (5).

11. Implant selon l'une des revendications précédentes, **caractérisé en ce qu'**au moins la couche extérieure du corps creux est réalisée dans un matériau sélectionné entre un polymère à mémoire de forme ou un matériau gonflant.

12. Implant selon l'une des revendications précédentes, **caractérisé en ce que** l'implant est composé d'un polymère biodégradable et/ou non biodégradable.

13. Implant selon la revendication 12, **caractérisé en ce que** les couches sont composées de matériaux polymères identiques et/ou différents, les matériaux polymères étant sélectionnés parmi des polymères biodégradables et/ou non biodégradables.

14. Implant selon l'une des revendications précédentes, **caractérisé en ce que** l'implant ou différentes couches de l'implant sont composés d'au moins un polymère sélectionné parmi des poly(alpha-hydroxy esters), des polyacrylates, un copolymère d'éthylène-acétate de vinyle ou un silicone.

15. Implant selon l'une des revendications précédentes, **caractérisé en ce que** l'agent actif est présent dans l'implant ou dans une couche de l'implant dans une teneur allant jusqu'à 30 % en poids.

16. Implant selon l'une des revendications précédentes, **caractérisé en ce que** la surface extérieure de l'implant présente des inégalités.

17. Implant selon l'une des revendications précédentes, **caractérisé en ce que** l'implant présente des zones complémentaires où le rapport q entre le diamètre extérieur et le diamètre intérieur est inférieur à 1,2 ou supérieur à 3.

18. Implant selon l'une des revendications précédentes, **caractérisé en ce que** le corticoïde comprend un glucocorticoïde, sélectionné dans le groupe composé du cortisol, de la cortisone, de la prednisone, de la prednisolone, de la 6-méthylprednisolone, de la dexaméthasone, de la fludrocortisone et de l'acétate de désoxycortone.

19. Implant selon l'une des revendications précédentes, où au moins une extrémité du corps creux est extensible après insertion dans le sinus.

20. Implant selon l'une des revendications précédentes, **caractérisé en ce que** l'implant est composé de matériau biodégradable, sélectionné dans le groupe des polymères et des copolymères de l'acide lactique et des polymères et des copolymères de l'acide glycolique.

21. Implant selon l'une des revendications précédentes, **caractérisé en ce que** l'implant est composé de matériau non biodégradable, sélectionné dans le groupe composé des silicones, polyacrylates, polyméthacrylates, copolymères de polyacrylates et de polyméthacrylates et copolymères poly(éthylène-acétate de vinyle).

FIG.1

FIG.2

FIG.3

FIG. 4

FIG. 5

FIG. 6

FIG. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5633000 A
- US 5019372 A
- US 4803076 A
- US 4069307 A
- US 3393073 A
- US 3967618 A
- US 4016251 A
- US 4052505 A
- US 3948254 A
- US 5693065 A
- US 5336163 A
- US 5601594 A

- US 5980551 A
- US 6080190 A
- US 5843089 A
- US 5824048 A
- US 5700286 A
- US 5837313 A
- US 5679400 A
- WO 9629071 A
- WO 9215286 A
- US 5139832 A
- US 5189110 A
- DE 4032096

**In der Beschreibung aufgeführte Nicht-Patentliteratur**

- **HOSEMANN WG ; WEBER RK ; KEERL RE ; LUND VJ.** Minimally invasive endonasal sinus surgery. Thieme, 2000
- **HOSEMANN W ; TH. KÜHNEL ; P. HELD, W. ; WAGNER, A.** Felderhoff: Endonasal frontal sinusotomy in surgical management of chronic sinusitis - a critical evaluation. *Am. J. Rhinology,* 1997, vol. 11, 1-9
- **DRAF W.** Endonasal micro-endoscopic frontal sinus surgery: the Fulda concept. *Op Tech Otolaryngol Head Neck Surg,* 1991, vol. 2, 234-240
- **MAY M ; SCHAITKIN B.** Frontal sinus surgery: endonasal drainage instead of an external osteoplastic approach. *Op Tech Otolaryngo Head Neck Surg,* 1995, vol. 6, 184-192
- **FLETSCHER INGALS E.** New operation and instruments for draining the frontal sinus. *Ann Otol Rhinol Laryngol,* 1905, vol. 14, 515-519
- **GOOD RH.** An intranasal method for opening the frontal sinus establishing the largest possible drainage. *Laryngoscope,* 1908, vol. 18, 266-274
- **STAMMBERGER H.** Komplikationen entzündlicher Nasennebenhöhlenerkrankungen einschließlich iatrogen bedingter Komplikationen. *Eur Arch Oto-Rhino-Laryngol,* 1993, vol. I, 61-186
- **JACOBS JB.** 100 years of frontal sinus surgery. *Laryngoscope,* 1997, vol. 107, 1-36
- **WEBER, R ; W. HOSEMANN ; W. DRAF ; R. KEERL ; B. SCHICK ; S. SCHINZEL.** Endonasale Stirnhöhlenchirurgie mit Langzeiteinlage eines Platzhalters. *Laryngol. Rhinol. Otol.,* 1997, vol. 76, 728-734

- **HOSEMANN, M.E. ; WIGAND, U. ; GÖDE, F. ; LÄNGER, I.** Dunker: Normal wound healing of the paranasal sinuses - clinical and experimental investigations. *Eur. Arch. Otorhinolaryngol.,* 1991, vol. 248, 390-394
- **HOSEMANN W ; GÖDE U ; LÄNGER F ; WIGAND ME.** Experimentelle Untersuchungen zur Wundheilung in den Nasennebenhöhlen. II. Spontaner Wundschluss und medikamentöse Effekte im standardisierten Wundmodell. *HNO,* 1991, vol. 39, 48-54
- **HOSEMANN W ; KÜHNEL TH ; ALLERT MH.** Weiterbehandlung nach Nasennebenhöhleneingriffen, Teil 2: Therapeutische Maßnahmen. *HNO aktuell,* 1999, vol. 7, 291-302
- **PRINCE MEP ; LEMCKERT RJ.** Analysis of the intranasal distribution of ointment. *J Otolaryngol,* 1997, vol. 26, 357-360
- **WEBER R ; KEERL R ; RADZIWILL R ; SCHICK B ; JASPERSEN D ; DSHAMBAZOV K ; MLYNSKI G ; DRAF W.** Videoendoscopic analysis of nasal steroid distribution. *Rhinology,* 1999, vol. 37, 69-73
- Hals-Nasen-Ohrenkrankheiten. **BUMM P.** Cortisontherapie, Corticoide in Klinik und Praxis. Thieme, 1992, 390 401
- **K. PARK ; W.S.W SHALABY ; H. PARK.** Biodegradable Hydrogels for Drug Delivery. Technomic Publishing Inc, 1993
- **A. DOMB ; J. KOST ; D.M. WISEMAN.** Handbook of Biodegradable Polymers. Harwood Academic Publishers, 1997

• **GÖPFERICH.** Polymer Degradation and Erosion: Mechanismus and Applications. *Eur. J. Pharm. Biopharm.,* 1996, vol. 42, 1-11